# EUROPEAN PATENT SPECIFICATION

(11) **EP 1 379 676 B1**
(45) Date of publication and mention of the grant of the patent: **24.05.2006**
(21) Application number: 02724247.8
(22) Date of filing: 19.03.2002
(51) Int. Cl.: C07D 207/12, C12P 7/26, C07H 15/12

(54) **PROCESS FOR THE PREPARATION OF 2-HYDROXYMETHYL-PYROLIDINE-3,4-DIOLS**
VERFAHREN ZUR HERSTELLUNG VON 2-HYDROXYMETHYL-PYROLIDIN-3,4-DIOLEN
PROCEDE DE PREPARATION DE 2-HYDROXYMETHYL-PYROLIDINE-3,4-DIOLS

(30) Priority: 19.03.2001 EP 01106794
(43) Date of publication of application: 14.01.2004
(73) Proprietor: Lonza AG, 4052 Basel (CH)
(72) Inventor: KÄMPFEN, Ulrich, CH-3900 Brig (CH); URBAN, Eva, Maria, CH-3912 Termen (CH); JESSEN, Claus, Ulrich, DK-2720 Vanl se (DK); CLAUSEN, Martin, CH-3900 Brig (CH)
(74) Representative: Reiss, Gilles François
(86) International application number: PCT/EP2002/003019
(87) International publication number: WO 2002/074737

(56) References cited:
- EP-A- 0 624 652
- JONES, J. K. N. ET AL: "The synthesis of acetamido deoxy ketoses by Acetobacter suboxydans. III" CAN. J. CHEM. (1962), 40, 503-10,1962, XP009000614
- HUNG, REBECCA R. ET AL: ".alpha.-Amino aldehyde equivalents as substrates for rabbit muscle aldolase: synthesis of 1,4-dideoxy-D-arabinitol and 2(R),5(R)- bis(hydroxymethyl)-3(R),4(R)-dihydroxypyrr olidine" JOURNAL OF ORGANIC CHEMISTRY (1991), 56(12), 3849-55,1991, XP001120216 cited in the application
- STANKOVIC L ET AL: "SUBSTITUTION OF HYDROGEN BY DEUTERIUM HINDRANCE OF THE BIOCHEMICAL DEHYDROGENATION OF ALDITOLS IN THE SENSE OF THE BERTRAND HUDSON RULE" CHEMICKE ZVESTI, vol. 34, no. 5, 1980, pages 695-699, XP009000619 ISSN: 0366-6352
- RAMIZ, A. ET AL: "N-Glycidyl(ityl)amides. Synthesis and mesophasic properties" COMUNICACIONES PRESENTADAS A LA JORNADAS DEL COMITE ESPANOL DE LA DETERGENCIA (2001), 31, 243-252, XP001120224

## Description

The present invention relates to a process for the preparation of 2-hydroxymethyl-pyrrolidine-3,4-diols, to a process for the preparation of *N*-formyl-5-amino-5-deoxy-pentuloses as well as to the compounds *N*-formyl-1-amino-1-deoxy-D-arabinitol and *N*-formyl-1-amino-1-deoxy-L-arabinitol.

Polyhydroxylated pyrrolidines constitute a class of glycosidase inhibitors. (2*R*,3*R*,4*R*)-2-hydroxymethyl-pyrrolidine-3,4-diol inhibits several α- and β-glucosidases and mannosidases with values of ID₅₀ below 10 µM (Hung *et al. J. Org. Chem.* **1991,** *56,* 3849-3855). Its enantiomer (2*S*,3*S*,4*S*)-2-hydroxymethyl-pyrrolidine-3,4-diol was shown to inhibit yeast α-glucosidase (Fleet *et al. Tetrahedron Lett.* **1985*****,** 26,* 3127-3130).

Fleet *et al. (Tetrahedron Lett.* **1985***, 26,* 3127-3130) describe the preparation of (2*R*,3*R*,4*R*)-and (2*S*,3*S*,4*S*)-2-hydroxymethyl-pyrrolidine-3,4-diol from D-xylose. The disadvantages of both syntheses are that they involve a large number of steps and that the overall yields are low.

Hung *et al.* (*J. Org. Chem.* **1991,** *56*, 3849-3855) describe the preparation of (2*R*,3*R*,4*R*)-2-hydroxymethyl-pyrrolidine-3,4-diol from 3-azido-propenyl-benzene. Ozonolysis of 3-azido-propenyl-benzene and subsequent enzymatically catalyzed aldol condensation of the obtained azido-acetaldehyde with dihydroxyacetone phosphate followed by dephosphorylation affords 5-azido-5-deoxy-D-xylulose, which is hydrogenated in the presence of palladium yielding (2*R*,3*R*,4*R*)-2-hydroxymethyl-pyrrolidine-3,4-diol. A disadvantage of the synthesis is that the hydrogenation is performed with a large amount of catalyst.

EP 0 624 652 A1 describes the preparation of 2-hydroxymethyl-pyrrolidine-3,4-diols and N-substituted derivatives thereof. 2-Hydroxymethyl-pyrrolidine-3,4-diol is prepared by microbial oxidation of *N*-benzyl-1-amino-1-deoxy-arabinitol using microorganisms of the genera *Gluconobacter* or *Corynebacterium* and subsequent hydrogenation using palladium as catalyst. *N*-Benzyl-1-amino-1-deoxy-arabinitol may be prepared from arabinose and benzylamine. The disadvantages of the preparation of 2-hydroxymethyl-pyrrolidine-3,4-diol are that the microbial oxidation affords the oxidation product in low volume yield and that the catalytic hydrogenation is performed with large amounts of catalyst.

It is an object of the present invention to provide an economic process for the preparation of 2-hydroxymethyl-pyrrolidine-3,4-diols. It is another object of the present invention to provide an economic process for the preparation of *N*-formyl-5-amino-5-deoxy-pentuloses and to provide new *N*-acylated 1-amino-1-deoxy-arabinitols.

These objects are achieved by the processes according to claims 1, 12, and 17 and by the compounds according to claims 22 and 23.

The process of the present invention for the preparation of 2-hydroxymethyl-pyrrolidine-3,4-diols comprises the steps of
a) oxidizing an *N*-protected aminotetraol of the formula 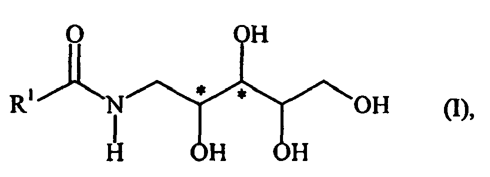 or a salt thereof, wherein R¹ is H, substituted or unsubstituted C₁₋₄-alkyl, substituted or unsubstituted C₂₋₄-alkenyl or OR², R² being unsubstituted C₁₋₄-alkyl, with a microorganism or a cell-free extract thereof to yield the corresponding *N*-protected 5-amino-5-deoxy-pentulose of the formula 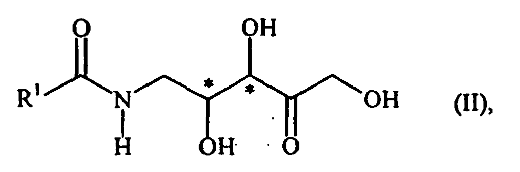 R¹ being defined as above,
b) removing the *N*-protective group of said *N*-protected 5-amino-5-deoxy-pentalose (II) to yield the corresponding 5-amino-5-deoxy-pentulose of the formula 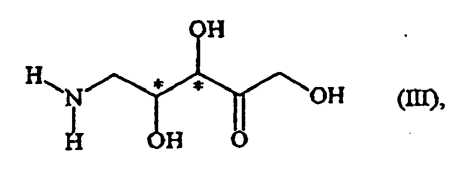 and
c) catalytically hydrogenating said 5-amino-5-deoxy-pentulose (III) to afford the corresponding (2*R*)- and/or (2*S*)-2-hydroxymethyl-pyrrolidine-3,4-diol of the formula

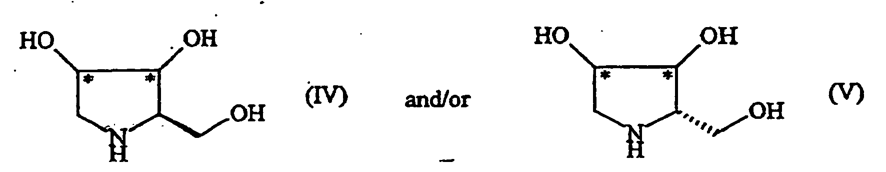

The asterisks in the formulae I, II, III, IV and V denote chiral carbon atoms with defined configuration.

*N*-Protected aminotetraols are selected from the group consisting of *N*-protected 1-amino-1-deoxy-D-arabinitols, *N*-protected 1-amino-1-deoxy-L-arabinitols, *N*-protected 1-amino-1-deoxy-D-ribitols, *N*-protected 1-amino-1-deoxy-L-ribitols, *N*-protected 1-amino-1-deoxy-D-xylitols, *N*-protected 1-amino-1-deoxy-L-xylitols and *N*-protected 1-amino-1-deoxy-D-lyxitols and *N*-protected 1-amino-1-deoxy-L-lyxitols.

Preferred *N*-protected aminotetraols are *N*-protected 1-amino-1-deoxy-D-arabinitols and *N*-protected 1-amino-1-deoxy-L-arabinitols, preferably *N*-formyl-1-amino-1-deoxy-D-arabinitols and *N-*formyl-1-amino-1-deoxy-L-arabinitols.

The more preferred *N*-protected aminotetraols are *N*-protected 1-amino-1-deoxy-D-arabinitols, preferably *N*-formyl-1-amino-1-deoxy-D-arabinitols.

Salts of *N*-protected aminotetraols are e.g. the salts formed by treating *N*-protected aminotetraols with strong mineral acids such as HCl.

The *N*-protected 5-amino-5-deoxy-pentulose which corresponds to *N*-protected 1-amino-1-deoXy-L-ribitol and *N*-protected 1-amino-1-deoxy-o-lyxitol is *N*-protected 5-amino-5-deoxy-D-ribulose. The *N*-protected 5-amino-5-deoxy-pentulose which corresponds to *N*-protected 1-amino-1-deoxy-D-ribitol and *N*-protected 1-amino-l-deoxy-L-lyxitol is *N*-protected 5-amino-5-deoxy-L-ribulose. The *N*-protected 5-amino-5-deoxy-pentulose which corresponds to *N*-protected 1-amino-1-deoxy-L-arabinitol and *N*-protected 1-amino-1-deoxy-L-xylitol is L-xylulose. The *N*-protected 5-amino-5-deoxy-pentulose which corresponds to *N*-protected 1-amino-1-deoxy-D-xylitol and *N*-protected 1-amino-1-deoxy-n-arabinitol is *N*-protected 5-amino-5-deoxy-D-xylulose.

The 5-amino-5-deoxy-pentulose which corresponds to *N*-protected 5-amino-5-deoxy-D-ribulose is 5-amino-5-deoxy-D-ribulose. Accordingly, 5-amino-5-deoxy-L-ribulose corresponds to *N*-protected 5-amino-5-deoxy-L-ribulose, 5-amino-5-deoxy-L-xylulose corresponds to *N*-protected 5-amino-5-deoxy-L-xylulose and 5-amino-5-deoxy-D-xylulose corresponds to *N*-protected 5-amino-5-deoxy-D-xylulose.

The (2*R*)- and/or (2*S*)-2-hydroxymethyl-pyrrolidine-3,4-diol which correspond to 5-amino-5-deoxy-D-ribulose are (2*R*,3*S*,4*R*)- and/or (2*S*,3*S*,4*R*)-2-hydroxymethyl-pyrrolidine-3,4-diol. Accordingly, (2*R*,3*R*,4*S*)- and/or (2*S*,3*R*,4*S*)-hydroxymethyl-pyrrolidine-3,4-diol correspond to 5-amino-5-deoxy-L-ribulose, (2*R*,3*S*,4*S*)- and/or (2*S*,3*S*,4*S*)-2-hydroxymethyl-pyrrolidine-3,4-diol correspond to 5-amino-5-deoxy-L-xylulose and (2*R*,3*R*,4*R*)- and/or (2*S*,3*R*,4*R*)-2-hydroxymethyl-pyrrolidine-3,4-diol correspond to 5-amino-5-deoxy-D-xylulose.

C₁₋₄-Alkyl may be branched or unbranched and may be substituted with at least one hydroxyl group and/or halogen atom. Halogen atoms may be fluorine, chlorine, bromine or iodine. Examples of unsubstituted C₁₋₄-alkyl are methyl, ethyl, propyl, isopropyl, butyl, isobutyl and *tert*-butyl. The corresponding *N*-protective groups for R¹ being unsubstituted C₁₋₄-alkyl are acetyl, propanoyl, butanoyl, isobutanoyl, pentanoyl, isopentanoyl and 2,2-dimethylpropanoyl (pivaloyl). The corresponding *N*-protective groups for R¹ being OR² and R² being unsubstituted C₁₋₄-alkyl are methoxycarbonyl, ethoxycarbonyl, isopropoxycarbonyl, butoxycarbonyl, isobutoxycarbonyl and *tert*-butoxycarbonyl. Examples of substituted C₁₋₄-alkyl are chloromethyl, dichloromethyl, trichloromethyl, fluoromethyl, difluoromethyl, trifluoromethyl, hydroxymethyl and 1-hydroxy-ethyl. The corresponding *N*-protective groups for R¹ being substituted C₁₋₄-alkyl are chloroacetyl, dichloroacetyl, trichloroacetyl, fluoroacetyl, difluoroacetyl, trifluoroacetyl, hydroxyacetyl and 2-hydroxy-propanoyl.

C₂₋₄-Alkenyl may be branched or unbranched and may be substituted with at least one hydroxyl group and/or halogen atom. Halogen atoms may be fluorine, chlorine, bromine or iodine. Examples for unsubstituted C₂₋₄-alkenyl are ethenyl, 1-propenyl, 2-propenyl, 1-methyl-2-propenyl, 2-methyl-1-propenyl, 1-butenyl, 2-butenyl and 3-butenyl. The corresponding *N*-protective groups for R¹ being unsubstituted C₂₋₄-alkenyl are acryloyl, crotonoyl, 3-butenoyl, 2-methyl-3-butenoyl, 3-methyl-2-butenoyl, 2-pentenoyl, 3-pentenoyl and 4-pentenoyl. Examples for substituted C₂₋₄-alkenyl are 1-chloroethenyl and 2-chloroethenyl. The corresponding *N*-protective groups for R¹ being substituted C₂₋₄-alkenyl are 2-chloroacryloyl and 3-chloroacryloyl.

For R¹ being H, the corresponding *N*-protective group is formyl.

Preferably, R¹ is H or substituted or unsubstituted C₁₋₂-alkyl. More preferably, R¹ is H.

The formyl protective group has the advantage that it provides a high solubility of the *N*-protected aminotetraol in aqueous medium. Thus the biooxidation can be performed at high concentrations of *N*-formyl aminotetraol affording a high volume yield of *N*-formyl-5-amino-5-deoxy-pentulose. In the biooxidation of *N*-formyl-1-amino-1-deoxy-D-arabinitol volume yields of *N*-formyl-5-amino-5-deoxy-D-xylulose of up to 200 g/L can be achieved.

The *N*-protected aminotetraols can be prepared from the corresponding aminotetraol by procedures known to a person skilled in the art.
*N*-Formyl-aminotetraols (R¹ being H) may be prepared from the corresponding aminotetraol by the reaction of the aminotetraol with an alkyl formate such as methyl formate, ethyl formate or butyl formate, with an aryl formate such as phenyl formate, with a mixed anhydride of formic acid and another carboxylic acid such as acetic formic anhydride or with formic acid.
*N*-Acyl-aminotetraols (R¹ being substituted or unsubstituted C₁₋₄-alkyl or substituted or unsubstituted C₂₋₄-alkenyl) may be prepared by the reaction of the aminotetraol with the appropriate acyl chloride, carboxylic acid alkyl ester, carboxylic acid aryl ester, carboxylic acid anhydride or carboxylic acid. N-Acetyl aminotetraols may be prepared from the corresponding aminotetraol by reaction with acetyl chloride, acetic anhydride or acetic acid, for example.
*N*-Alkoxycarbonyl-aminotetraols (R¹ being OR² and R² being unsubstituted C₁₋₄-alkyl) may be prepared by reacting the corresponding aminotetraol with the appropriate alkyl chloroformate, alkyl azidoformate or with the appropriate dialkyl-dicarbonate.
*N*-*tert*-Butoxycarbonyl-aminotetraols may be prepared by reacting the corresponding aminotetraol with di-tert-butyl dicarbonate, for example.

The aminotetraol may be prepared from the corresponding pentoses such as D-arabinose, L-arabinose, D-xylose, L-xylose, D-ribose, L-ribose, D-lyxose and L-lyxose by procedures known to a person skilled in the art. The pentose may be reacted with a primary or secondary amine or with hydroxylamine to the corresponding imine or oxime, which may be catalytically hydrogenated to afford the aminotetraol. 1-Amino-1-deoxy-D-arabinitol may be prepared from D-arabinose and 1-amino-1-deoxy-L-arabinitol may be prepared from L-arabinitol by reacting the appropriate enantiomer of arabinose with benzylamine under reducing conditions and catalytically hydrogenating the obtained corresponding N-benzyl-1-amino-1-deoxy-arabitinol.

The biooxidation step can be performed with any microorganism capable of oxidizing the hydroxyl group at C-4 of the *N*-protected aminotetraols to a keto group. The microorganism may be a bacterium or a fungus. Suitable bacteria are of the genera *Gluconobacter, Acetobacter* or *Corynebacterium.* Suitable fungi are of the genera *Metschnikowia, Candida* or *Saccharomyces.*

Preferred microorganisms are bacteria of the genera *Gluconobacter* or *Acelobacter.*

More preferred microorganisms are of the species *Gluconobacter oxydans.* Bacteria of the species *Gluconobacter oxydans* shall also refer to the subspecies *Gluconobacter oxydans ssp. suboxydans* (formerly known as *Acetobacter suboxydans* or *Acetomonas suboxydans)* and *Gluconobacter oxydans ssp. oxydans* (formerly known as *Acetomonas oxydans).*

Most preferred microorganisms are selected from the group of strains consisting of *Gluconobacter oxydans ssp. suboxydans* with the designations DSM 2003 (DSM 14076), DSM 2343 and DSM 50049 (ATCC 621).

These strains can be obtained from the Deutsche Sammlung für Zelikulturen und Mikroorganismen GmbH (DSMZ), Mascheroder Weg 1b, D-38124 Braunschweig, Germany. The strain *Gluconobacter oxydans ssp. suboxydans* DSM 2003 was deposited on 23.02.2001 at the DSMZ under the terms of the Budapest Treaty with the designation DSM 14076.

The bacterial strains *Gluconobacter oxydans ssp. suboxydans* with the designations DSM 2003 (DSM 14076), DSM 2343 and DSM 50049 (ATCC 621) shall also refer to mutants thereof belonging to the species *Gluconobacter oxydans ssp. suboxydans* and being able to oxidize the hydroxyl group at C-4 of the *N*-protected aminotetraol to a keto group. Such mutants may be obtained by induced or spontaneous mutation of the above bacterial strains, followed by the isolation of the obtained mutants and screening of the isolated mutants for their ability to oxidize the hydroxyl group at C-4 of the *N*-protected aminotetraol to a keto group. Examples for mutations are substitution, insertion or deletion of single or multiple bases or inversion of DNA segments of the genome. Spontaneous mutations are naturally occurring mutations due to errors in DNA replication. Induced mutants may be obtained by procedures known to a person skilled in the art such as by radiation (ultraviolet radiation, X-rays or gamma rays), mutagenic chemicals (ethyl methanesulfonate, nitrite or bromouracil) or by polymerase chain reaction (PCR).

The microorganisms may be grown on suitable media comprising nutrients, which serve as carbon, nitrogen and energy sources, such as soya peptone and yeast extract, and an inducer of the enzymatic activity catalyzing the oxidation of the hydroxyl group at C-4 of the *N*-protected aminotetraols to a keto group. Suitable inducers of the enzymatic activity are sugar alcohols such as D-mannitol, L-mannitol, D-sorbitol, L-sorbitol, galactitol, erythritol, D-threitol and L-threitol. Preferred inducers are D-sorbitol and L-sorbitol. The more preferred inducer is D-sorbitol.

The growth of the microorganisms may be performed at pH 5.0-8.0, preferably at pH 5.5-7.0, and at 20-35°C, preferably at 28-32°C.

Preferably, the growth is performed under aerobic conditions. Precultures may be grown in flasks having a gas permeable lid and fermentations may be performed in fermenters or stirred vessels under gassing with air or oxygen, for example. The fermentation may be performed at ambient pressure (ca. 1 bar) or at overpressure up to 3 bar. More preferably, the growth is performed while keeping the amount of oxygen in the fermentation broth above 60% (P_{O²}/P_{O²sat.} at atmospheric pressure), most preferably above 70%, by gassing with air.

After growth the microorganisms can be harvested by e.g. filtration or centrifugation.

The biooxidation of the *N*-protected aminotetraol to the *N*-protected-1-amino-1-deoxy-pentulose is carried out with the microorganisms or with cell extracts thereof. The microorganisms may be employed as resting cells, as immobilized resting cells or as growing cells. Cell extracts thereof refer to the crude cell extracts which are obtained by microbial cell disruption methods known to a person skilled in the art or by autolysis. Examples of microbial cell disruption procedures are ultrasound or French press. Preferably, the biooxidation is carried out with a microorganism, more preferably with resting cells of a microorganism.

Preferably, the concentration of the *N*-protected aminotetraol is 50-250 g/L, more preferably 100-250 g/L, most preferably 150-250 g/L.

Preferably, the biooxidation is performed at pH 4.3-6.0, more preferably at pH 4.5-5.5, and at 10-50°C, more preferably at 10-20°C.

The biooxidation may be carried out as a batch, fed-batch or as a continuous process.

The biooxidation is usually performed for 12-84 h, preferably for 12-36 h.

After biooxidation, the cells are removed by e.g. filtration or centrifugation. The obtained solution may be used directly in the next step or the *N*-protected 5-amino-5-deoxy-D-xylulose may be isolated from the solution before used in the next step.

The removal of the *N*-protective group of *N*-protected 5-amino-5-deoxy-pentulose is performed by hydrolysis under alkaline conditions or by using an enzyme such as an acylase.

Preferably, the *N*-protective group is removed under alkaline conditions. Examples of suitable bases are alkali metal hydroxides, alkaline earth metal hydroxides, ammonium hydroxide, dialkylammonium hydroxides, trialkylammonium hydroxides, tetraalkylammonium hydroxides, alkali metal carbonates or alkali earth metal carbonates. Examples of alkali metal hydroxides are sodium hydroxide, potassium hydroxide and lithium hydroxide. Examples of alkaline earth metal hydroxides are calcium hydroxide and barium hydroxide. An example of dialkylammonium hydroxides is diisopropylammonium hydroxide. An examples of trialkylammonium hydroxides is triethylammonium hydroxide. An example of tetraalkyl ammonium hydroxides is tetrabutyiammonium hydroxide.
Examples of alkali metal carbonates are sodium carbonate, potassium carbonate and lithium carbonate. Examples of alkaline earth metal carbonates are calcium carbonate and barium carbonate.

More preferably the *N*-protective group is removed with 1-2 mol equivalents of an alkali hydroxide in respect to *N*-protected 5-amino-5-deoxy-pentulose.

Most preferably the *N*-protective group is removed with 1-2 mol equivalents of sodium hydroxide or potassium hydroxide in respect to *N*-protected 5-amino-5-deoxy-pentulose.

Suitable hydrogenation catalysts include nickel or noble metals such as palladium, rhodium or platinum as the reducing agent. Examples of hydrogenation catalysts are *Raney* nickel, palladium on charcoal, palladium on barium sulfate, palladium on calcium carbonate, platinum on charcoal, rhodium on charcoal and rhodium on aluminium oxide. The noble metal content of the catalyst is 1-20% (w/w), preferably 4-10% (w/w). Preferably, the hydrogenation catalyst is a palladium catalyst, more preferably the hydrogenation catalyst is palladium on charcoal.

The amount of catalyst used in the hydrogenation may be 0.1-100% (weight of catalyst/weight of 5-amino-5-deoxy-pentulose), preferably 0.5-20%, more preferably 0.5-10%.

The solvent used in the hydrogenation may be water or a water-miscible alcohol such as methanol, ethanol, propanol or isopropanol or mixtures of water and a water-miscible alcohol, preferably water or mixtures of water and a water-miscible alcohol, more preferably water.

The hydrogenation may be carried out at 0-120°C, preferably, at 15-40°C, more preferably at 20-30°C, and at a hydrogen pressure of 0.5-100 bar, preferably of 2-10 bar, more preferably at 4-6 bar.

The reaction time for hydrogenation is usually 1-50 h, preferably 5-40 h, more preferably 10-30 h.

The hydrogenation usually provides either the (2*R*)- or the (2*S*)-2-hydroxymethyl-pyrrolidine-3,4-diol in high diastereomeric excess (d.e.), preferably >80% d.e., more preferably >90% d.e..

After the hydrogenation, the catalyst can be removed by e.g. filtration. The product 2-hydroxymethyl-pyrrolidone may be purified by ion exchange chromatography.

The removal of the *N*-protective group and the catalytic hydrogenation can be separate process steps or can be performed in the same process step. Preferably the removal of the *N*-protective group and the catalytic hydrogenation are performed in the same process step.

Also part of the present invention is a process for the preparation of 2-hydroxymethyl-3,4-diol comprising the steps of
a) removing the *N*-protective group of *N*-protected 5-amino-5-deoxy-pentulose of the formula 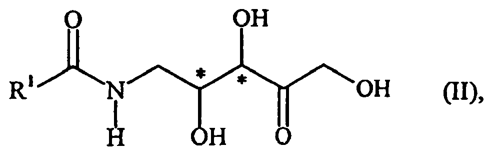 to yield the corresponding 5-amino-5-deoxy-pentulose of the formula 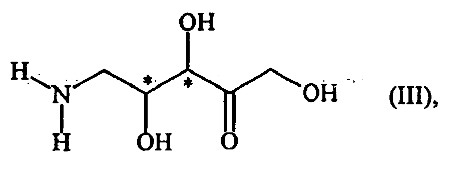 and
b) catalytically hydrogenating said 5-amino-5-deoxy-pentulose (III) to produce the corresponding (2*R*)- and/or (2*S*)-hydroxymethyl-pyrrolidine-3,4-diol of the formula

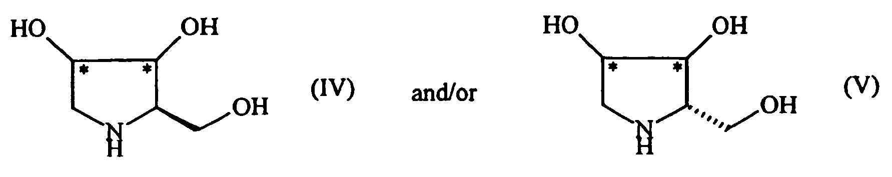

Suitable *N*-protected 5-amino-5-deoxy-pentuloses are *N*-protected 5-amino-5-deoxy-D-xylulose, *N*-protected 5-amino-5-deoxy-L-xylulose, *N*-protected 5-amino-5-deoxy-D-ribulose and *N*-protected 5-amino-5-deoxy-L-ribulose. Preferred *N*-protected 5-amino-5-deoxy-pentuloses are *N*-protected 5-amino-5-deoxy-D-xylulose and *N*-protected 5-amino-5-deoxy-L-xylulose. A more preferred *N*-protected 5-amino-5-deoxy-pentulose is 5-amino-5-deoxy-D-xylulose.

All definitions given for the preparation of 2-hydroxymethyl--pyrrolidine-3,4-diol from *N*-protected aminotetraol (I) apply accordingly, when appropriate, to this process.

### Example 1

### Preparation of N-formyl-1-amino-1-deoxy-D-arabinitol

1-Amino-1-deoxy-D-arabinitol (50.0 g, 330 mmol) was dissolved in methanol (1300 mL) by warming up to 35°C. After cooling to 20°C, methyl formate (43.6 g, 725 mmol) was added and the solution was stirred at ca. 20°C in a double walled flask with mechanical stirrer and heating-cooling system. After 2 h a white solid precipitated. After a reaction time of overall 3 h, the mixture was cooled to 0°C in 1 h under stirring. For work up, the obtained white suspension was filtered off and washed twice with cold methanol (35 mL each). Drying of the precipitate at 40°C and 25 mbar afforded *N*-formyl-1-amino-1-deoxy-D-arabitinol (82%).

### Example 2

### Preparation of N-formyl-1-amino-1-deoxy-L-arabinitol

The formylation was carried out as described in example 1, except that 1-amino-1-deoxy-L-arabinitol was used as starting material and *N*-formyl-1-amino-1-deoxy-L-arabitinol was obtained.

### Example 3

### Biomass preparation

Medium A was prepared by dissolving yeast extract (3 g/L), D-sorbitol (60 g/L) and soya peptone (5 g/L) in deionized water and adjusting the pH to 6.3 ± 0,3 with NaOH. Medium A was placed in 3 Erlenmeyer flasks (100 mL each), sterilized (121°C, 20 min, 1 bar) and inoculated with *Gluconobacter oxydans ssp. suboxydans* DSM 2003 (DSM 14076) (1 mL each). The precultures were incubated at 30°C and 200 rpm for 24 h. Medium A (11.5 L) was placed in a 30 L fermentor, sterilized (121°C, 30 min, 1 bar) and inoculated with preculture (200 mL). The fermentation was performed at 28°C, at pH 6.3 keeping the amount of oxygen in the fermentation broth above 70% (P_{O²}/P_{O² sat.} at atmospheric pressure) by gassing with air. After ca. 13 h the fermentation broth was cooled and filtrated at 4°C. The cells were washed by ultrafiltration with aqueous MgSO₄ (20 mM) and concentrated. The concentrated cell suspension was diluted with aqueous MgSO₄ (20 mM) to OD₆₅₀ₙₘ 200 and stored at 4°C.

### Example 4

### Biooxidation of N-formyl-1-amino-1-deoxy-D-arabinitol

*N*-Formyl-1-deoxy-1-amino-D-arabinitol (20 g, 111 mmol) was dissolved in deionized water (100 mL). Concentrated cell suspension of *Gluconobacter oxydans ssp. suboxydans* DSM 2003 (DSM 14076) prepared as described in example 3 was added and the pH was adjusted to 5.0. The biooxidation was performed at 15°C and pH 5 under shaking. After 24 h the starting material was almost quantitatively converted to *N*-formyl-5-amino-5-deoxy-D-xylulose as determined by TLC. The cells were removed by ultrafiltration and the obtained solution was stored until hydrogenation.

### Example 5

### Biooxidation of N-formyl-1-amino-1-deoxy-L-arabinitol

The biooxidation was carried out as described in example 4, except that *N*-formyl-1-amino-1-deoxy-L-arabinitol was used as starting material. N-Formyl-1-amino-1-deoxy-L-arabinitol was converted to *N*-formyl-5-amino-5-deoxy-L-xylulose.

### Example 6

### Catalytic hydrogenation of N-formyl-5-amino-5-deoxy-D-xylulose

An aqueous solution of *N*-formyl-5-amino-5-deoxy-D-xylulose (111 mmol) obtained as described in example 4, KOH (1 M, 111 mL, 111 mmol) and Pd/C (4% with 53.4 % water, Degussa-Hüls, 1.0 g) were mixed in deionized water (50 mL) in a Hastelloy® 1 L autoclave with heating-cooling system. The reaction mixture was purged three times with nitrogen (5 bar) and then three times with H₂ (5 bar). The reaction vessel was pressurized with H₂ (5 bar). After 20 h stirring at 23°C, the reaction mixture was purged three times with N₂. After filtration over Celite®, a brownish solution (183 g) was obtained. The solution was purified with an ion exchange resin column (diameter 3 cm, height 20 cm, Dowex® 50Wx8H, 100-200 mesh). The resin was activated with aqueous HCl (7%) and then washed with water to a pH of 3-4. After product loading the column was purged 5 times with water (168 mL each time), then eluted with aqueous NH₃ (5%, 114 mL) and at the end washed with water (283 mL). The fractions were collected and checked with TLC (solvent system: dichloromethanelwater/aqueous ammonia (25%): 17/3/2, detection: KMnO₄/Na₂CO₃: 1/1): R_{*f*} ((2*R*,3*R*,4*R*)-2-hydroxymethyl-pyrrolidine-3,4-diol)- 0.2-0.4, R_{f} (*N-*formyl-5-amino-5-deoxy-D-xylulose): 0.83). After pooling of identical fractions and removal of water, (2*R*,3*R*,4*R*)-2-hydroxymethyl-pyrrolidine-3,4-diol (72% starting from *N*-formyl-1-amino-1-deoxy-D-arabinitol) with a diastereomeric excess of 94.4% as determined by high pressure capillary electrophoresis (HPCE) was obtained.

### Example 7

### Catalytic hydrogenation of N-formyl-5-amino-5-deoxy-L-xylulose

The hydrogenation was carried out as described in example 6, except that *N*-formyl-5-amino-5-deoxy-L-xylulose was used as starting material. (2*S*,3*S*,4*S*)-2-hydroxymethyl-pyrrolidine-3,4-diol was obtained.

### Example 8

### Removal of N-formyl protective group prior to catalytic hydrogenation

An aqueous solution of *N*-formyl-5-amino-5-deoxy-D-xylulose (100 g, 111 mmol) obtained as described in example 4, KOH (1 M, 111 mL, 111 mmol) and deionized water (50 mL) were stirred in a Hastelloy® 1 L autoclave with heating-cooling system for 1 h at ca. 23°C.

Pd/C (4% with 53.4 % water (Degussa-Hüls), 1.0 g) was added and the hydrogenation was performed as described in example 6. (2*R*,3*R*,4*R*)-2-hydroxymethyl-pyrrolidine-3,4-diol was obtained.

## Claims

1. Process comprising the steps of
a) removing the *N*-protective group of *N*-protected 5-amino-5-deoxy-pentulose of the formula 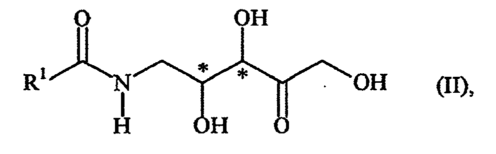 wherein R¹ is H, substituted or unsubstituted C₁₋₄-alkyl, substituted or unsubstituted C₂₋₄-alkenyl or OR², R² being unsubstituted C₁₋₄-alkyl, under alkaline conditions to yield the corresponding 5-amino-5-deoxy-pentulose of the formula 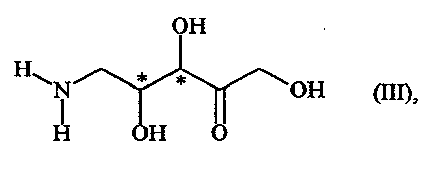 and
b) catalytically hydrogenating said 5-amino-5-deoxy-pentulose (III) to produce the corresponding (2*R*)- and/or (2*S*)-2-hydroxymethyl-pyrrolidine-3,4-diol of the formula

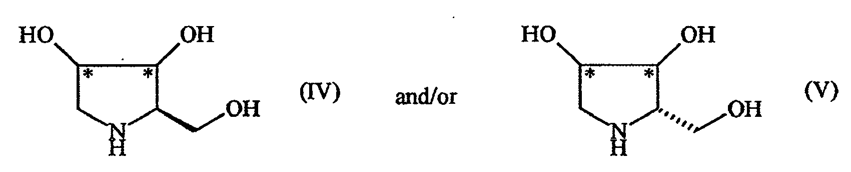

2. Process according to claim 1, **characterized in that** the *N*-protected 5-amino-5-deoxy-pentulose is *N*-protected 5-amino-5-deoxy-D-xylulose or *N*-protected 5-amino-5-deoxy-L-xylulose.

3. Process according to claim 2, **characterized in that** the *N*-protected 5-amino-5-deoxy-pentulose is *N*-protected 5-amino-5-deoxy-D-xylulose.

4. Process according to one of the preceding claims, **characterized in that** the *N-*protective group is removed with 1-2 molar equivalents of an alkaline hydroxide in respect to the 5-amino-5-deoxy-pentulose.

5. Process according to one of the preceding claims, **characterized in that** the hydrogenation catalyst is a palladium catalyst.

6. Process according to claim 5, **characterized in that** the amount of catalyst used in the hydrogenation is 0.5-20% (weight of catalyst/weight of 5-amino-5-deoxy-pentulose).

7. Process according to one of the preceding claims, **characterized in that** the removal of the *N*-protective group and the catalytic hydrogenation are carried out in the same process step.

8. A process comprising the steps of
a) oxidizing an *N*-protected aminotetraol of the formula 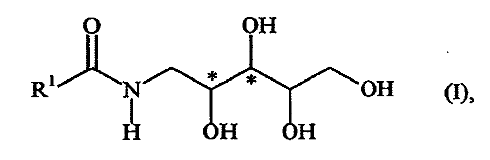 or a salt thereof wherein R¹ is H, substituted or unsubstituted C₁₋₄-alkyl, substituted or unsubstituted C₂₋₄-alkenyl or OR², R² being unsubstituted C₁₋₄-alkyl, with a microorganism or a cell-free extract thereof to yield the corresponding *N*-protected 5-amino-5-deoxy-pentulose of the formula 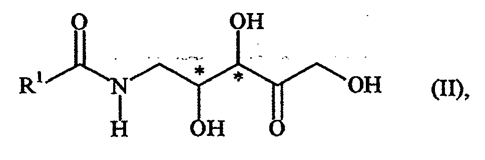 R¹ being defined as above,
b) removing the *N*-protective group of said *N*-protected 5-amino-5-deoxy-pentulose (II) under alkaline conditions to yield the corresponding 5-amino-5-deoxy-pentulose of the formula 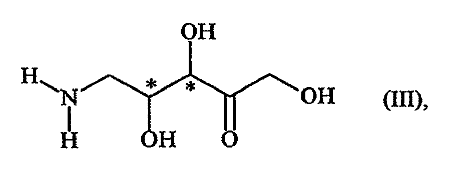 and
c) catalytically hydrogenating said 5-amino-5-deoxy-pentulose (III) to produce the corresponding (2*R*)- and/or (2*S*)-hydroxymethyl-pyrrolidine-3,4-diol of the formula

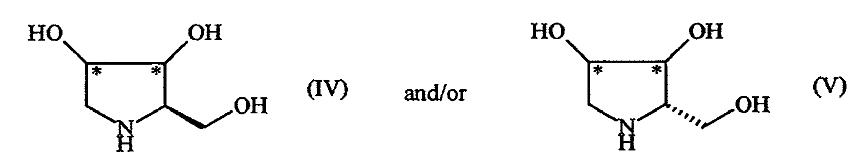

9. Process according to one of claims 1-8, **characterized in that** R¹ is substituted or unsubstituted C₁₋₄-alkyl or H.

10. Process according to claim 9, **characterized in that** R¹ is C₁₋₂-alkyl or H.

11. Process according to claim 10, **characterized in that** R¹=H.

12. Process according to claim 8, **characterized in that** the *N*-protected aminotetraol is an *N*-protected 1-amino-1-deoxy-L-arabinitol or an *N*-protected 1-amino-1-deoxy-D-arabinitol.

13. Process according to one of claims 8-12, **characterized in that** the microorganism belongs to the genera *Gluconobacter* or *Acetobacter,* preferably to the species *Gluconobacter oxydans.*

14. Process according to claim 13, **characterized in that** the microorganism is selected from the group of strains consisting of *Gluconobacter oxydans ssp. suboxydans* DSM 2003 (DSM 14076), *Gluconobacter oxydans ssp. suboxydans* DSM 2343 and *Gluconobacter oxydans ssp. suboxydans* DSM 50049 (ATCC 621).

15. Process according to one of claims 8-14, **characterized in that** the oxidation is carried out at a concentration of *N*-protected aminotetraol of 50-250 g/L, preferably at 100-250 g/L.

16. Process according to one of claims 8-15, **characterized in that** the oxidation is carried out at pH 4.3-6.0 and at 10-50°C.

17. Process according to one of claims 9-16 , **characterized in that** the *N*-protective group is removed with 1-2 mol equivalents of an alkaline hydroxide in respect to the *N-*protected 5-amino-5-deoxy-pentulose.

18. Process according to one of claims 9-16, **characterized in that** the hydrogenation catalyst is a palladium catalyst and wherein the amount of catalyst used in the hydrogenation is 0.5-10% (weight of catalyst/weight of 5-amino-5-deoxy-pentulose).

19. Process according to one of claims 1-18, **characterized in that** the removal of the *N*-protective group and the catalytic hydrogenation are carried out in the same process step.

20. *N*-Formyl-1-amino-1-deoxy-L-arabinitol of the formula

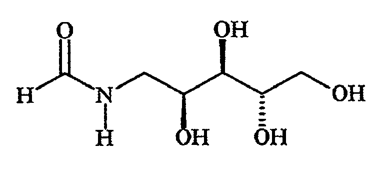

21. *N*-Formyl-1-amino-1-deoxy-D-arabinitol of the formula

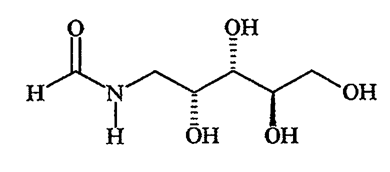

## Patentansprüche

1. Verfahren, bei dem man
a) die *N*-Schutzgruppe einer *N*-geschützten 5-Amino-5-desoxypentulose der Formel 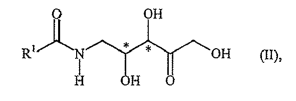 in welcher R¹ für H, substituiertes oder unsubstituiertes C₁₋₄-Alkyl, substituiertes oder unsubstituiertes C₂₋₄-Alkenyl oder OR² steht, wobei R² für unsubstituiertes C₁₋₄-Alkyl steht, unter alkalischen Bedingungen entfernt, was die entsprechende 5-Amino-5-desoxypentulose der Formel 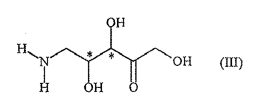 liefert, und
b) diese 5-Amino-5-desoxypentulose (III) katalytisch zum entsprechenden (2*R*)- und/oder (2*S*)-2-Hydroxymethylpyrrolidin-3,4-diol der Formel 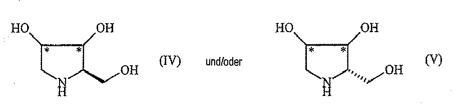 hydriert.

2. Verfahren nach Anspruch 1, **dadurch gekennzeichnet, daß** es sich bei der *N-*geschützten 5-Amino-5-desoxypentulose um *N*-geschützte 5-Amino-5-desoxy-D-xylulose oder *N*-geschützte 5-Amino-5-desoxy-L-xylulose handelt.

3. Verfahren nach Anspruch 2, **dadurch gekennzeichnet, daß** es sich bei der *N-*geschützten 5-Amino-5-desoxypentulose um *N*-geschützte 5-Amino-5-desoxy-D-xylulose handelt.

4. Verfahren nach einem der vorhergehenden Ansprüche, **dadurch gekennzeichnet, daß** die *N*-Schutzgruppe mit 1-2 Moläquivalenten eines alkalischen Hydroxids, bezogen auf die 5-Amino-5-desoxypentulose, entfernt wird.

5. Verfahren nach einem der vorhergehenden Ansprüche, **dadurch gekennzeichnet, daß** es sich bei dem Hydrierungskatalysator um einen Palladiumkatalysator handelt.

6. Verfahren nach Anspruch 5, **dadurch gekennzeichnet, daß** die bei der Hydrierung verwendete Menge an Katalysator 0,5-20% (Gewicht des Katalysators/ Gewicht der 5-Amino-5-desoxypentulose) beträgt.

7. Verfahren nach einem der vorhergehenden Ansprüche, **dadurch gekennzeichnet, daß** das Entfernen der *N*-Schutzgruppe und die katalytische Hydrierung im gleichen Verfahrensschritt durchgeführt werden.

8. Verfahren, bei dem man
a) ein *N*-geschütztes Aminotetraol der Formel 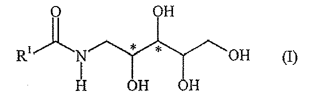 oder ein Salz davon, wobei R¹ für H, substituiertes oder unsubstituiertes C₁₋₄-Alkyl, substituiertes oder unsubstituiertes C₂₋₄₋Alkenyl oder OR² steht, wobei R² für unsubstituiertes C₁₋₄-Alkyl steht, mit einem Mikroorganismus oder einem zellfreien Extrakt davon zur entsprechenden *N*-geschützten 5-Amino-5-desoxypentulose der Formel 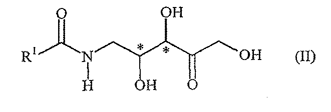 oxidiert, wobei R¹ wie oben definiert ist,
b) die *N*-Schutzgruppe der *N*-geschützten 5-Amino-5-desoxypentulose (II) unter alkalischen Bedingungen entfernt, was die entsprechende 5-Amino-5-desoxypentulose der Formel 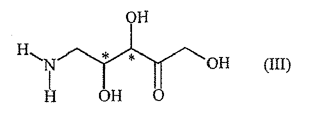 liefert, und
c) diese 5-Amino-5-desoxypentulose (III) katalytisch zum entsprechenden (2*R*)- und/oder (2*S*)-Hydroxymethylpyrrolidin-3,4-diol der Formel 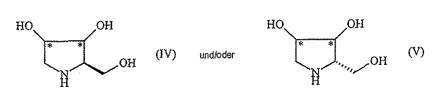 hydriert.

9. Verfahren nach einem der Ansprüche 1-8, **dadurch gekennzeichnet, daß** R¹ für substituiertes oder unsubstituiertes C₁₋₄-Alkyl oder H steht.

10. Verfahren nach Anspruch 9, **dadurch gekennzeichnet, daß** R¹ für C₁₋₂-Alkyl oder H steht.

11. Verfahren nach Anspruch 10, **dadurch gekennzeichnet, daß** R¹ = H.

12. Verfahren nach Anspruch 8, **dadurch gekennzeichnet, daß** es sich bei dem *N*-geschützten Aminotetraol um ein *N*-geschütztes 1-Amino-1-desoxy-L-arabinitol oder ein *N*-geschütztes 1-Amino-1-desoxy-D- arabinitol handelt.

13. Verfahren nach einem der Ansprüche 8-12, **dadurch gekennzeichnet, daß** der Mikroorganismus zu der Gattung *Gluconobacter* oder *Acetobacter,* vorzugsweise zur Spezies *Gluconobacter oxydans*, gehört.

14. Verfahren nach Anspruch 13, **dadurch gekennzeichnet, daß** der Mikroorganismus ausgewählt ist aus der aus *Gluconobacter oxydans ssp. suboxydans* DSM 2003 (DSM 14076), *Gluconobacter oxydans ssp. suboxydans* DSM 2343 und *Gluconobacter oxydans ssp. suboxydans* DSM 50049 (ATCC 621) bestehenden Gruppe von Stämmen.

15. Verfahren nach einem der Ansprüche 8-14, **dadurch gekennzeichnet, daß** die Oxidation bei einer Konzentration an *N*-geschütztem Aminotetraol von 50-250 g/l, vorzugsweise bei 100-250 g/l, durchgeführt wird.

16. Verfahren nach einem der Ansprüche 8-15, **dadurch gekennzeichnet, daß** die Oxidation bei einem pH-Wert von 4,3-6,0 und bei 10-50°C durchgeführt wird.

17. Verfahren nach einem der Ansprüche 9-16, **dadurch gekennzeichnet, daß** die *N*-Schutzgruppe mit 1-2 Moläquivalenten eines alkalischen Hydroxids, bezogen auf die *N*-geschützte 5-Amino-5-desoxypentulose, entfernt wird.

18. Verfahren nach einem der Ansprüche 9-16, **dadurch gekennzeichnet, daß** es sich bei dem Hydrierungskatalysator um einen Palladiumkatalysator handelt und die Menge an bei der Hydrierung verwendetem Katalysator 0,5-10% (Gewicht des Katalysators/ Gewicht der 5-Amino-5-desoxypentulose) beträgt.

19. Verfahren nach einem der Ansprüche 1-18, **dadurch gekennzeichnet, daß** die Entfernung der *N*-Schutzgruppe und die katalytische Hydrierung im gleichen Verfahrensschritt durchgeführt werden.

20. *N*-Formyl-1-amino-1-desoxy-L- arabinitol der Formel

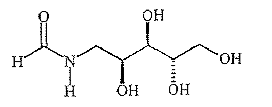

21. *N*-Formyl-1-amino-1-desoxy-D- arabinitol der Formel

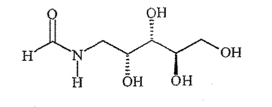

## Revendications

1. Procédé comprenant les étapes consistant à
a) éliminer le groupe *N*-protecteur d'un 5-amino-5-désoxy-pentulose *N-*protégé de formule 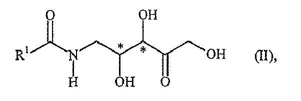 dans laquelle R¹ est un H, un alkyle en C₁₋₄ substitué ou non substitué, un alcényle en C₂₋₄ substitué ou non substitué ou un OR², R² étant un alkyle en C₁₋₄ non substitué, dans des conditions alcalines pour donner lieu au 5-amino-5-désoxy-pentulose correspondant de formule 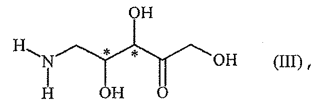 et
b) hydrogéner catalytiquement ledit 5-amino-5-désoxy-pentulose (III) pour produire le (2*R*)- et/ou le (2*S*)-2-hydroxyméthyl-pyrrolidine-3,4-diol correspondant de formule

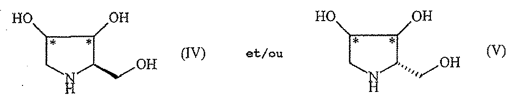

2. Procédé selon la revendication 1, **caractérisé en ce que** le 5-amino-5-désoxy-pentulose *N*-protégé est un 5-amino-5-désoxy-D-xylulose *N-*protégé ou un 5-amino-5-désoxy-L-xylulose *N*-protégé.

3. Procédé selon la revendication 2, **caractérisé en ce que** le 5-amino-5-désoxy-pentulose *N*-protégé est un 5-amino-5-désoxy-D-xylulose *N-*protégé.

4. Procédé selon l'une des revendications précédentes, **caractérisé en ce que** le groupe *N*-protecteur est éliminé avec 1-2 équivalents molaires d'un hydroxyde alcalin par rapport au 5-amino-5-désoxy-pentulose.

5. Procédé selon l'une des revendications précédentes, **caractérisé en ce que** le catalyseur d'hydrogénation est un catalyseur au palladium.

6. Procédé selon la revendication 5, **caractérisé en ce que** la quantité de catalyseur utilisée dans l'hydrogénation est de 0,5 à 20% (poids de catalyseur/poids de 5-amino-5-désoxy-pentulose).

7. Procédé selon l'une des revendications précédentes, **caractérisé en ce que** l'élimination du groupe *N*-protecteur et l'hydrogénation catalytique sont effectuées dans la même étape de procédé.

8. Procédé comprenant les étapes consistant à
a) oxyder un aminotétraol *N*-protégé de formule 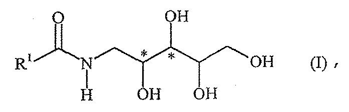 ou un sel de celui-ci, dans laquelle R¹ est un H, un alkyle en C₁₋₄ substitué ou non substitué, un alcényle en C₂₋₄ substitué ou non substitué ou un OR², R² étant un alkyle en C₁₋₄ non substitué, avec un micro-organisme ou un extrait de celui-ci exempt de cellules, pour donner lieu au 5-amino-5-désoxy-pentulose *N*-protégé de formule 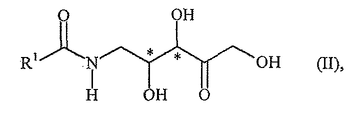 R¹ étant défini comme ci-dessus,
b) éliminer le groupe *N*-protecteur dudit 5-amino-5-désoxy-pentulose *N-*protégé (II) dans des conditions alcalines pour donner lieu au 5-amino-5-désoxy-pentulose correspondant de formule 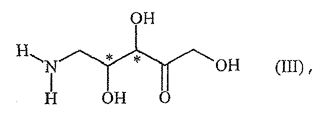 et
b) hydrogéner catalytiquement ledit 5-amino-5-désoxy-pentulose (III) pour produire le (2*R*)- et/ou le (2*S*)-2-hydroxyméthyl-pyrrolidine-3,4-diol correspondant de formule

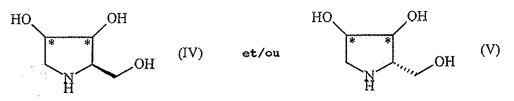

9. Procédé selon l'une des revendications 1 à 8, **caractérisé en ce que** R¹ est un alkyle en C₁₋₄ substitué ou non substitué ou un H.

10. Procédé selon la revendication 9, **caractérisé en ce que** R¹ est un alkyle en C₁₋₂ ou un H.

11. Procédé selon la revendication 10, **caractérisé en ce que** R¹ est un H.

12. Procédé selon la revendication 8, **caractérisé en ce que** l'aminotétraol *N-*protégé est un 1-amino-1-désoxy-L-arabinitol *N*-protégé ou un 1-amino-1-désoxy-D-arabinitol *N*-protégé.

13. Procédé selon l'une des revendications 8 à 12, **caractérisé en ce que** le micro-organisme appartient aux genres *Gluconobacter* ou *Acétobacter,* de préférence à l'espèce *Gluconobacter oxydans.*

14. Procédé selon la revendication 13, **caractérisé en ce que** le micro-organisme est choisi parmi le groupe de souches constitué de *Gluconobacter oxydans ssp. Suboxydans* DSM 2003 (DSM 14076), de *Gluconobacter oxydans ssp. Suboxydans* DSM 2343 et de *Gluconobacter oxydans ssp. Suboxydans* DSM 50049 (ATCC 621).

15. Procédé selon l'une des revendications 8 à 14, **caractérisé en ce que** l'oxydation est effectuée à une concentration en aminotétraol *N*-protégé de 50 à 250 g/l, de préférence de 100 à 250 g/l.

16. Procédé selon l'une des revendications 8 à 15, **caractérisé en ce que** l'oxydation est effectuée à un pH de 4,3 à 6,0 et entre 10 et 50°C.

17. Procédé selon l'une des revendications 9 à 16, **caractérisé en ce que** le groupe *N*-protecteur est éliminé avec 1-2 équivalents molaires d'un hydroxyde alcalin par rapport au 5-amino-5-désoxy-pentulose *N*-protégé.

18. Procédé selon l'une des revendications 9 à 16, **caractérisé en ce que** le catalyseur d'hydrogénation est un catalyseur au palladium et où la quantité de catalyseur utilisée dans l'hydrogénation est de 0,5 à 10 % (poids de catalyseur/poids de 5-amino-5-désoxy-pentulose).

19. Procédé selon l'une des revendications 1 à 18, **caractérisé en ce que** l'élimination du groupe *N*-protecteur et l'hydrogénation catalytique sont effectuées dans la même étape de procédé.

20. *N*-Formyl-1-amino-1-désoxy-L-arabinitol de formule

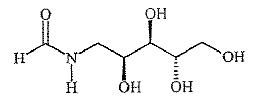

21. *N*-Fomlyl-1-anlino-1-désoxy-D-arabinitol de formule
